(19) Europäisches Patentamt
European Patent Office
Office européen des brevets

(11) Veröffentlichungsnummer : **0 499 922 B1**

(12) # EUROPÄISCHE PATENTSCHRIFT

(45) Veröffentlichungstag der Patentschrift : **06.07.94 Patentblatt 94/27**

(51) Int. Cl.$^5$ : **C07C 39/16,** C07C 39/17, C07C 37/86

(21) Anmeldenummer : **92102152.3**

(22) Anmeldetag : **10.02.92**

(54) **Verfahren zur Reinigung von Bisphenolen.**

(30) Priorität : **21.02.91 DE 4105428**

(43) Veröffentlichungstag der Anmeldung : **26.08.92 Patentblatt 92/35**

(45) Bekanntmachung des Hinweises auf die Patenterteilung : **06.07.94 Patentblatt 94/27**

(84) Benannte Vertragsstaaten : **BE DE FR GB IT NL**

(56) Entgegenhaltungen :
**EP-A- 0 319 326**
**EP-A- 0 332 203**
**EP-A- 0 343 349**
**FR-A- 2 073 717**
**US-A- 4 180 683**
**PATENT ABSTRACTS OF JAPAN vol. 5, no. 120 (C-65)(792) 4. August 1981**

(73) Patentinhaber : **BAYER AG**
**D-51368 Leverkusen (DE)**

(72) Erfinder : **Jakob, Wolfgang, Dip.-Ing.**
**Am Domacker 81**
**W-4130 Moers (DE)**
Erfinder : **Schmidt, Manfred, Dr.**
**Erich-Klausener-Strasse 37**
**W-4150 Krefeld (DE)**
Erfinder : **Freitag, Dieter, Dr.**
**Hasenheide 10**
**W-4150 Krefeld (DE)**
Erfinder : **Berg, Klaus D., Dr.**
**Hansastrasse 124**
**W-4150 Krefeld (DE)**

EP 0 499 922 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Reinigung von Bisphenolen durch Destillation.

Bekanntlich werden Bisphenole z.B. aus Ketonen und Phenolen in Gegenwart von Katalysatoren hergestellt (z.B. BE-PS 738 962, JP-A 62/178 534).

Das gebildete Bisphenol kann sich nach beendeter Reaktion als 1:1 Addukt mit dem jeweils verwendeten Phenol abscheiden. Dieses Addukt (Mischkristall) kann zur weiteren Reinigung mit dem jeweils verwendeten Phenol gewaschen werden. Zur Spaltung der Mischkristalle kann das Phenol mit Lösungsmitteln extrahiert werden oder der Mischkristall wird erschmolzen und aus der Schmelze bei hoher Temperatur das Phenol abdestilliert. Für die Extraktion muß dann der Extrakt aufwendig aufgearbeitet werden. Das Abdestillieren von Phenol aus den Mischkristallen oberhalb des Schmelzpunktes der Mischkristalle bei hohen Temperaturen, kann ungünstig, z.B. in Bezug auf das hergestellte Bisphenol, sein.

Es wurde nun gefunden, daß aus Bisphenol/Phenol-Addukten, wie sie bei bekannten Verfahren zur Herstellung von Bisphenolen anfallen, das Phenol unterhalb des Schmelzpunktes des Bisphenol/Phenol-Adduktes (Mischkristall) bei vermindertem Druck entfernt werden kann.

Gegenstand der Erfindung ist daher ein Verfahren zur Reinigung von Bisphenolen, die bei bekannten Verfahren zu ihrer Herstellung aus Ketonen und Phenolen als Bisphenol/Phenol-Addukte anfallen, dadurch gekennzeichnet, daß das Phenol bei Temperaturen unterhalb des Schmelzpunktes des Bisphenol/Phenol-Adduktes bei vermindertem Druck entfernt wird.

Vorzugsweise können erfindungsgemäß Bisphenole der Formel (I)

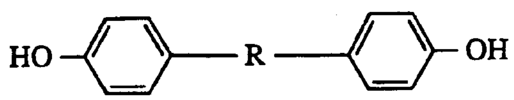 (I),

in welcher

R für $C_1$-$C_{10}$ Alkylen und $C_5$-$C_{15}$-Cycloalkylen steht,

eingesetzt werden.

Besonders bevorzugte Bisphenole sind beispielsweise Bisphenol A (2,2-Bis[-4-hydroxiphenyl]-propan), Bisphenol TMC (1,1-Bis[4-hydroxiphenyl]3,3,5-trimethylcyclohexan), Bisphenol Z (1,1-Bis-(4-hydroxiphenyl)-cyclohexan), Bisphenol-3M-(1,1-Bis-(4-hydroxyphenyl)-3-methylcyclohexan).

Die Bisphenol/Phenol-Addukte (Mischkristalle) enthalten 0,1 bis 2,15 mol Phenol pro Mol Bisphenol. Vorzugsweise liegen die Mischkristalle etwa als 1:1 Addukte von Bisphenol/Phenol vor.

Erfindungsgemäß werden die Mischkristalle in der Festphase bei Temperaturen unterhalb ihres Schmelzpunktes gespalten. Vorzugsweise werden sie im Temperaturbereich von 50 bis 200°C, besonders bevorzugt im Bereich von 90-120°C, gespalten. Die Spaltungstemperatur liegt in den genannten Bereichen vorzugsweise 10°C unterhalb des Schmelzpunktes des jeweils eingesetzten Mischkristalls.

Die erfindungsgemäße Spaltung (bei der das Phenol absublimiert und das Bisphenol zurückbleibt), wird vorzugsweise ohne Lösungsmittel durchgeführt bei einem Druck von 0,5 bis 100 mbar, vorzugsweise 0,5-15 mbar.

Zur Durchführung der erfindungsgemäßen Reinigung der Bisphenole (Spaltung der Mischkristalle) weden übliche Trockner, z.B. Teller-, Tammel-, Schaufel-, Riesel-, Gegenstromtrockner usw. verwendet.

### Beispiele

Aus den Beispielen, die in der Tabelle 1 zusammegefaßt sind, erkennt man, daß sowohl das abgespaltene Phenol als auch das verbleibende Bisphenol aus der Festphasenmischkristallspaltung von größerer Reinheit sind als die korrespondierenden Produkte der Spaltung oberhalb des Schmelzpunktes.

Die Festphasentrocknung wurde in einem üblichen Laborschaufeltrockner durchgeführt, die Spaltung oberhalb des Schmelzpunktes in einer laborüblichen Destillationsapparatur. Die Destillate und die Rückstände wurden gaschromatographisch untersucht und/oder und ihre Farbzahl wurde bestimmt (Hazen Farbzahl nach DIN 53 409).

### Versuchsdurchführung:

A) 100 g der jeweiligen Mischkristalle wurden in einem 250 ml Dreihalskolben mit Brücke und Vorlage unter Vakuum (18 mbar) auf 180°C erhitzt und bei 180°C 30 min gehalten. Dabei destillierte Phenol ab.

B) 150 g der jeweiligen Mischkristalle wurden in einen Schaufeltrockner gefüllt. Es wurde ein Vakuum von ca. 18 mbar eingestellt und auf die Temperaturen, die aus der Tabelle ersichtlich sind, aufgeheizt. Dabei destillierte das Phenol ab.

Untersucht wurden

1) Mischkristalle aus Phenol und
 1,1-Bis-(4-hydroxiphenyl)-cyclohexan(BP-Z*).
2) Mischkristalle aus Phenol und
 1,1-Bis-(4-hydroxiphenyl)-3-methylcyclohexan (BP-3M*)
3) Mischkristalle aus Phenol und
 1,1-Bis-(4-hydroxiphenyl)-3,3,5-trimethylcyclohexan (BP-TMC*)
4) Mischkristalle aus Phenol und
 2,2-Bis-(4-hydroxiphenyl)-propan (BP-A)

*BP=Bisphenol

**Tabelle 1**

| Beispiel-Nr. | Produkt | T (°C) | Druck (mbar) | Zeit (min) | FZ[1)] (HZ) | GC-Reinheit [2)] (Gew.-%) | Verunreinigungen[3)] (Gew.-%) |
|---|---|---|---|---|---|---|---|
| 1,A | BPZ | 190 | 20 | 30 | 15-20 | 95,0 | 0,6 |
| | Phenol | | | | | 98,7 | 0,05 |
| 1,B | BPZ | 130 | 20 | 270 | 40 | 99,3 | 0,04 |
| | Phenol | | | | | 98,3 | 0,01 |
| 2,A | BP3M | 190 | 20 | 30 | grau | 97,1 | 1,5 |
| | Phenol | | | | | 99,6 | 0,18 |
| 2,B | BP3M | 130 | 20 | 180 | 20-30 | 98,9 | 1,0 |
| | Phenol | | | | | 99,7 | 0,17 |
| 3,A | BPTMC | 190 | 20 | 30 | 200 | 95,3 | 3,5 |
| | Phenol | | | | | 99,0 | 0,3 |
| 3,B | BPTMC | 130 | 20 | 240 | 40 | 99,9 | 0,01 |
| | Phenol | | | | | 97,8 | 0,01 |
| 4,A | BPA | 190 | 20 | 30 | 20 | 99,1 | 0,9 |
| | Phenol | | | | | 99,1 | 0,9 |
| 4,B | BPA | 90 | 20 | 180 | 15 | 99,7 | 0,3 |
| | Phenol | | | 90 | | 99,9 | 0,1 |

1) Farbzahl (FZ) des resultierenden Bisphenols bestimmt nach Hazen Farbzahl DIN 53 409
2) GC-Reinheit des Bisphenols oder des abdestillierten Phenols
3) andere als Bisphenol oder Phenol

## Patentansprüche

1. Verfahren zur Reinigung von Bisphenolen, die bei bekannten Verfahren zu ihrer Herstellung aus Ketonen und Phenolen als Bisphenol/Phenol-Addukte anfallen, dadurch gekennzeichnet, daß das Phenol bei Temperaturen unterhalb des Schmelzpunktes des Bisphenol/Phenol-Adduktes bei vermindertem Druck entfernt wird.

## Claims

1. A process for the purification of bisphenols accumulating as bisphenol/phenol adducts in known processes for their production from ketones and phenols, characterized in that the phenol is removed under reduced pressure at temperatures below the melting point of the bisphenol/ phenol adduct.

## Revendications

1. Procédé de purification de bisphénols, qui se forment en tant que produits d'addition bisphénolphénol lors des procédés connus pour leur fabrication à partir de cétones et de phénols, caractérisé en ce que le phénol est séparé à des températures inférieures au point de fusion du produit d'addition bisphénol/phénol à pression réduite.